# EUROPEAN PATENT APPLICATION

(11) **EP 3 196 597 A1**
(43) Date of publication of application: **26.07.2017**
(21) Application number: 16152235.4
(22) Date of filing: 21.01.2016
(51) Int. Cl.: G01D 3/10

(54) **A SYSTEM AND METHOD FOR LINKING OSCILLATING MOVEMENTS OF EXERCISE EQUIPMENT TO A USER OF THE EXERCISE EQUIPMENT IN A DATABASE**

(71) Applicant: Sony Mobile Communications, Inc., Minato-ku Tokyo 108-0075 (JP)
(72) Inventor: Bengtsson, Henrik, 227 36 Lund (SE); Fletcher, Christer, 244 95 Dösjebro (SE); Linge, Anders, 244 36 Kävlinge (SE); Nyman, Johan, 222 40 Lund (SE)
(74) Representative: Awapatent AB

(57) **Abstract**

The invention relates to a system comprising a first device (102) adapted to be attached to a user (100) and comprising a user ID, and a second device (112) adapted to be attached to a exercise equipment (112) and comprising an equipment ID. The system is arranged for linking oscillating movements of the exercise to the user of the exercise equipment in a database.

## Description

### Technical field

The invention relates generally to a system and a method for linking oscillating movements of an exercise equipment to a user of the exercise equipment in a database.

### Background

The market for connected devices, e.g. internet of things, is increasing. The areas of use are continuously developing. Connected devices may facilitate automation of activities that previously were done manually by a person. One such activity that may be automated is the activity of registering training activities of a person. For example, a smart phone with a suitable application may be used for measuring e.g. distance and speed of a running workout, and storing such data for later retrieval.

In a gym environment or similar, such automatic registration of exercise activities requires knowledge of which user is using which exercise equipment. One solution is to employ a log on system where the user logs on to the exercise equipment that he/she is using and then logs of when he/she has finished the exercise. However, this may be difficult for a user to remember while exercising. An alternative solution would thus be beneficial.

### Summary

In view of the above, an objective of the invention is to solve or at least reduce one or several of the drawbacks discussed above. Generally, the above objective is achieved by the attached independent patent claims.

According to a first aspect, the present invention is realized by a system for linking oscillating movements of an exercise equipment having a equipment ID to a user of the exercise equipment in a database, the system comprising:
- a first device adapted to be attached to a user, the first device comprising a user ID, the first device being arranged for extracting data pertaining to an oscillating movement of the first device,
- a second device adapted to be attached to the exercise equipment, the second device comprising the equipment ID, the second device being arranged for extracting data pertaining to an oscillating movement of the second device,
- a third device connected to the database and arranged for:
   o receiving said data pertaining to an oscillating movement of the first device, the user ID, the equipment ID and said data pertaining to an oscillating movement of the second device,
   o calculating a correlation between said data pertaining to an oscillating movement of the first device and said data pertaining to an oscillating movement of the second device, comparing the calculated correlation to a first threshold, and if the calculated correlation exceeds the first threshold, linking said oscillating movement of the exercise equipment to the user ID in the database using the equipment ID.

By the term "oscillating movements" should, in the context of present specification, be understood a repeating movement or a periodic motion, back and forth over a range of positions. Such a movement may for example be when the user is stretching and retracting his arms over his head, e.g. when doing a shoulder press exercise with a dumbbell.

By calculating a correlation between an oscillating movement of a first device attached to a user, and an oscillating movement of a second device attached to an exercise equipment (e.g. attached to a part of an exercise machine such as a rowing machine, to a dumbbell or to a barbell), the oscillating movement of the exercise equipment may be linked to the user wearing the first device. Consequently, the repetitions performed by the user on the exercise equipment may be registered to the user in a database. The training activities of the user may thus be automatically registered, and the user may focus on the training instead remember to manually print down and log each exercise. Moreover, with the present system, the user does not need to log on to the equipment that he/she intends to use.

In a second aspect, the present invention provides a method for linking oscillating movements of an exercise equipment to a user of the exercise equipment in a database, the method being performed in a system comprising a first device adapted to be attached to a user and comprising a user ID, a second device adapted to be attached to the exercise equipment and comprising an equipment ID of the exercise equipment, and a third device connected to the database, the method comprising the steps of:
- extracting, by the first device, data pertaining to an oscillating movement of the first device,
- extracting, by the second device, data pertaining to an oscillating movement of the second device,
- calculating, by the third device, a correlation between said data pertaining to an oscillating movement of the first device and said data pertaining to an oscillating movement of the second device,
- comparing, by the third device, the calculated correlation to a first threshold, and,
- if the calculated correlation exceeds the first threshold, linking, by the third device, said oscillating movement of the exercise equipment to the user ID in the database using the equipment ID.

The second aspect may generally have the same features and advantages as the first aspect.

Other objectives, features and advantages of the present invention will appear from the following detailed disclosure, from the attached dependent claims as well as from the drawings.

Generally, all terms used in the claims are to be interpreted according to their ordinary meaning in the technical field, unless explicitly defined otherwise herein. All references to "a/an/the [element, device, component, means, step, etc]" are to be interpreted openly as referring to at least one instance of the element, device, component, means, step, etc., unless explicitly stated otherwise.

### Brief description of drawings

Examples of embodiments herein are described in more detail with reference to attached drawings where the same reference numerals will be used for similar elements, wherein:
figure 1 shows a user wearing a first device and a second device attached to a exercise equipment according to embodiments of the invention,
figure 2 shows a user performing a exercise activity,
figure 3 shows a first and/or a second device according to embodiments of the invention,
figure 4 shows a system for linking oscillating movements of an exercise equipment to a user of the exercise equipment in a database according to a first embodiment,
figure 5 shows a system for linking oscillating movements of an exercise equipment to a user of the exercise equipment in a database according to a second embodiment,
figure 6 shows data of sensors arranged for measuring movements of the first and second device,
figure 7 shows data sent to the third device and data stored in the database which links an oscillating movements of an exercise equipment to a user according to embodiments of the invention,
figure 8 shows a method for linking oscillating movements of an exercise equipment to a user of the exercise equipment in a database according to embodiments of the invention.

### Detailed description

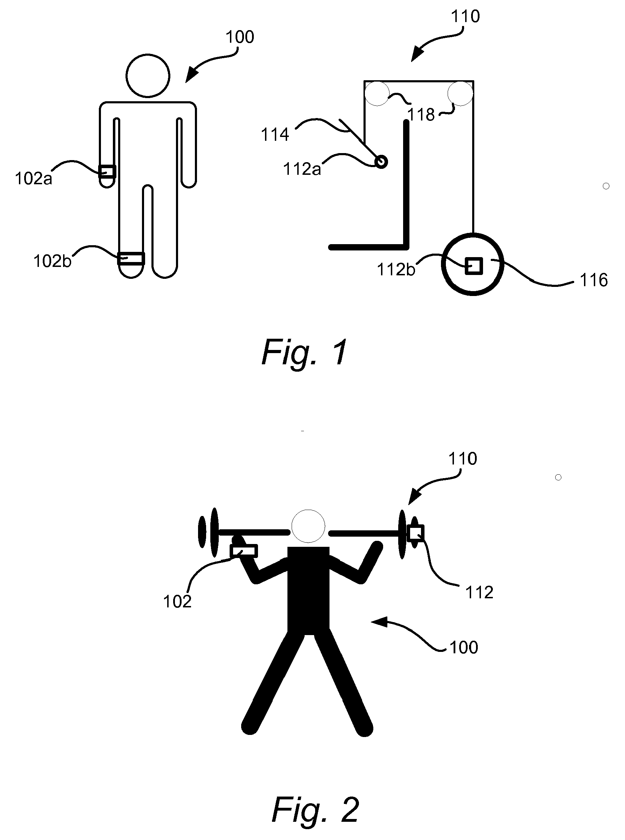

Figure 1 shows by way of example a person 100. A first device 102a is attached to the wrist of the person. A further first device 102b is attached to the foot of the person. These placements of the first devices 102a-102b is merely by way of example, other placements are equally possible. It should also be noted that according to some embodiments, only one, or more than two first devices 102 is/are attached to the user. By having a plurality of devices attached to the person, different exercises may be logged using the invention, since e.g. some exercises involves movement of the arms of the person 100 while others involve movement of the legs.

Figure 1 further shows an exercise equipment 110, in this case an exercise machine 110. The exercise machine 110 comprises a handle 114 for pulling a weight 116 up and down. The exercise machine 110 comprises a second device 112a-b, positioned on a part of the machine 110 which while the machine 110 is in use, will be oscillating. The second device 112a-b may in this example be positioned on the handle 114, and/or on the weight 116. Other positions are equally possible, for example on the wire connecting the handle 114 to the weight 116, or on one of the wheels 118 of the exercise machine 110.

The first 102a-b and second devices 112a-b are both arranged for extracting data pertaining to an oscillating movement of the respective device. The devices 102a-b, 112a-b may for example comprise accelerometers, or gyroscopes. This will be further described below.

Figure 2 shows a person 100 (user etc.) which is exercising using an exercise equipment 110 (in this case a barbell 110). Since the user 100 is wearing a first device 102 on the wrist and a second device 112 is attached to the barbell 110, the data extracted by the first and second device 102, 112 pertaining to an oscillating movement of the respective devices will be similar, and a correlation calculation between the two data should yield a high value.

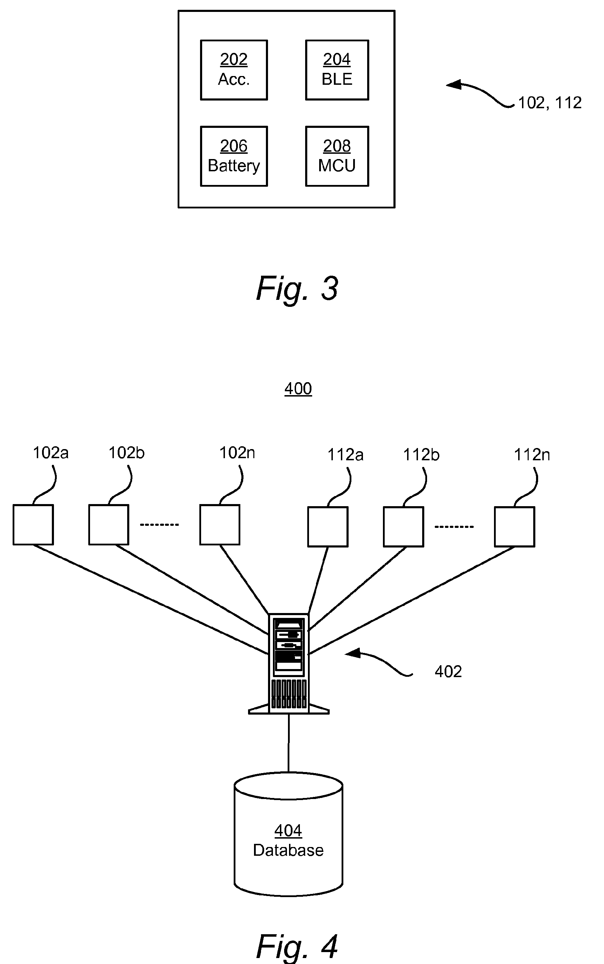

Figure 3 describe by way of example the design of a first 102 and second 112 device. The device 102, 112 may comprise a sensor 202 arranged for measuring movements of the device 102, 112 and for producing movement data pertaining to the measured movements. The sensor may for example be an accelerometer or a gyroscope. The accelerometer may be of any suitable type, e.g. an optical or a piezoelectric accelerometer.

The device 102 may further comprise a battery 206 for powering the device. According to some embodiments, it is important that the second device 112 (arranged to be attached to the exercise equipment) has a low power consumption, e.g. such that the battery can last for more than a year. This reduces the need of recharging/replacing the battery. In order to achieve this, the sensor 202 of the second device 112 may be arranged to have a first and a second state. The first state may be a low energy state where the sensor 202 is running at the lowest possible power and only arranged to detect a movement exceeding a threshold. When such a movement is detected, the sensor 202 may in response thereto configure itself in a second state in which the sensor 202 is arranged for measuring movements of the second device 202 and for producing movement data pertaining to the measured movements. Using this setup, the sensor may run at 10 microAmps when being in its first state, which with a 1000 mAh battery means 100 000 hours of battery time when the device is only in the first state. The battery time will obviously decrease when the sensor is in the second state, but generally, an exercise equipment is only used for a minor part of the day, and not used at all during the night. In some embodiments, when the sensor 202 has not detected any movement for a threshold amount of time, the sensor 202 will configure itself in the first state again.

Such a setup may not be needed in the first device 102 since this device generally may be recharged after each workout session and the battery 206 of the first device 102 thus only need to last for e.g. 3 hours. Of course, in order to reduce the need of recharging, also the sensor 202 of the first device 102 may be arranged as described above with a first and second state.

The device 102, 112 may further comprise a microcontroller or microcontroller unit (MCU) 208 arranged for receiving the movement data from the sensor 202, and for extracting data pertaining to the oscillating movement of the device 102, 112. This data may then be transmitted from the device 102, 112 by using a Bluetooth Low Energy (BLE) radio 204. Other ways of transmitting the data are equally possible such as regular Bluetooth, Wi-Fi, Cellular data service such as 3g/4g etc. It is also possible that the devices 112 attached to the exercise equipment transmit the data via a wired connection, especially if they are attached to an exercise machine which generally is not moved around in a gym environment.

The data transmitted from the BLE radio (or similar) 204 of the devices 102, 112 are then received by a third device 402 which is connected to a database 404. An example of this setup is shown in figure 4. In this example, the third device, e.g. a server, 402 is connected (wireless or wired as described above) to a plurality of first devices 102a-n and a plurality of second devices 112a-n. When receiving data from the devices 102a-n, 112 a-n, the third device 402 is arranged for calculating a correlation between data pertaining to an oscillating movement received from a first device 102a-n and data pertaining to an oscillating movement from a second device 112a-n in order to calculate which of the first devises 102a-n are attached to the user which causes a second device 112a-n to oscillate. This is done by calculating a correlation between data pertaining to an oscillating movement of a first device 102a-n and data pertaining to an oscillating movement of a second device 112a-n and comparing the calculated correlation to a threshold. In case the calculated correlation exceeds the threshold, it is determined by the third device 402 that the two oscillating movements are connected. Since the third device also is receiving a user ID from the first device 102a-n and the equipment ID, this user ID can be linked to the oscillating movement of the exercise equipment in the database 404. Consequently, an exercise activity of the exercise equipment having the equipment ID is automatically registered to the user having the user ID. The first device 102a-n may be configured with the user ID in any suitable way, e.g. by some central log in system at the gym, or by the user inputting a user name into the first device 102a-n when starting to use the first device 102a-n at the gym. Similarly, the second device 112a-n may be configured with the equipment ID in any suitable way such as configuring the second device with the equipment ID when attaching it to the exercise equipment.

The first device 102 a-b may for example be a smart phone, or a smart watch. The first device may according to some embodiments comprise an NFC-reader for configuring the first device with a user ID.

The second device 112a-b may be based on any type of data chip comprising the required features, and to which a sensor for measuring movements of the second device and for producing movement data pertaining to the measured movements can be connected. The data chip should also be easily connected to a power source, e.g. a battery. The chip should e.g. be suited for Bluetooth low energy and ultra low-power wireless applications and be built around a CPU with a flash memory and/or RAM memory for improved application performance. The chip should comprise a transmitter/transceiver which may support both BLE and/or Wi-Fi. Such a design may also be used for the first device 102 a-b.

The calculation of correlation performed by the third device may be carried out by a processor of the third device running software for calculating a correlation between two data sets.

In the embodiment shown in figure 4, the third device 402 is a separate device from the first and the second device. However, it is envisaged that the third device may also be part of the first or second device. In this embodiment, either the first or second device is connected to the database. The device that is connected to the database is further arranged to receive the data pertaining to an oscillating movement from the other device (i.e. first or second), calculate the correlation, and in case the correlation exceeds the first threshold, linking said oscillating movement of the exercise equipment to the user ID in the database using the equipment ID.

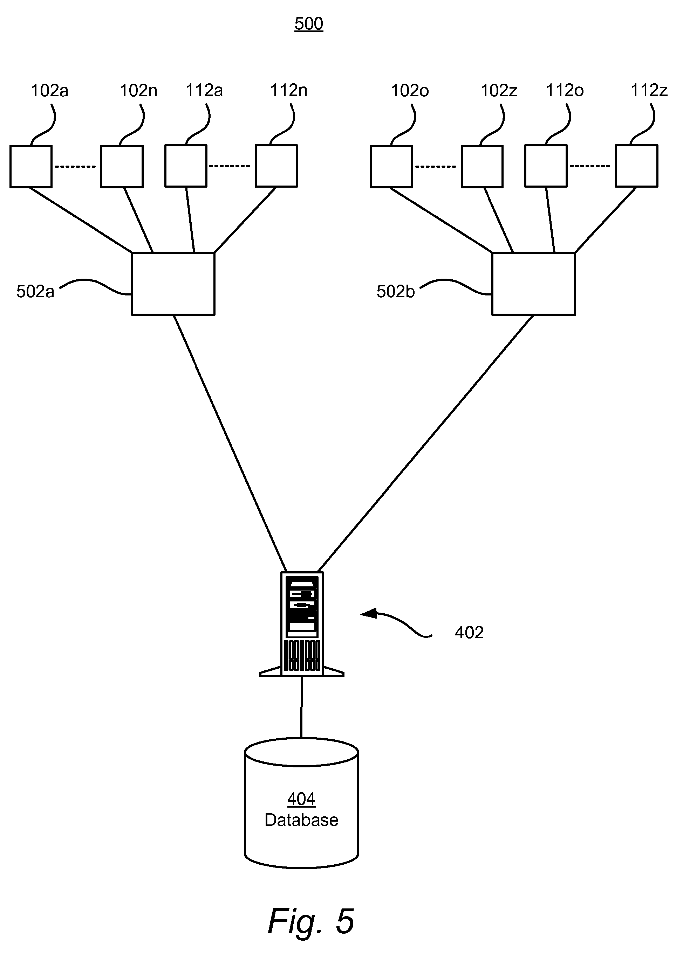

Figure 5 shows another possible setup, wherein the system 500 further comprises a fourth device 502a-b being wirelessly connected to the first and second device, and further being connected to the third device 402. This embodiment may be advantageous in the case where the third device 402 (server) is used for registering training activities for users for multiple gyms or locations.

The fourth devices 502a-b may thus be used for retransmitting the data received from the first and second devices of the gym where the fourth device is located, e.g. as shown in figure 5 where the fourth device 502a is connected (wireless or wired) to first devices 102a-n and second devices 112a-n while the fourth device 502b is connected (wireless or wired) to first devices 102o-z and second devices 112o-z.

According to some embodiments, a first device and a second device may be sending its data to more than one fourth device. For example if a single fourth device cannot cover the entire area of a gym, two or more fourth devices may be installed in the gym. In this case, the third device 402 may be configured to filter received data such that such duplicates will be removed. For example, if it receives data simultaneously from more than one fourth device, where more than one instance of data have the same user id or equipment id, the third device may use only one instance of data (of the more than one instance of data with the same user id or equipment id) when performing a correlation calculation and disregard the other instances.

According to some embodiments, the fourth device 502a-b may be any type of device which can receive data from the first/second device, e.g. equipped with a BLE/BT/Wi-Fi receiver and comprising a transmitter (e.g. WiFi) for retransmitting the data to the third device 412. The fourth device may for example be a laptop, or stationary computer. Other off the shelf-products such as an Intel Edison may be used, which according to one embodiment is connected to the first/second devices via Bluetooth (e.g. BLE) and connected to the third device 402 via Wi-Fi.

In order to reduce the risk of inadvertently logging a user id from one gym with an exercise equipment from another gym, the fourth device 502a-b may comprise an ID of a gym wherein the exercise equipment (i.e. the connected second devices) is located, wherein the fourth device is further arranged for transmitting the ID of the gym to the third device 402. This ID may then be used by the third device in a filtering step of the received data pertaining to an oscillating movement of the first devices/second device such that only data originating from the same gym may be correlated.

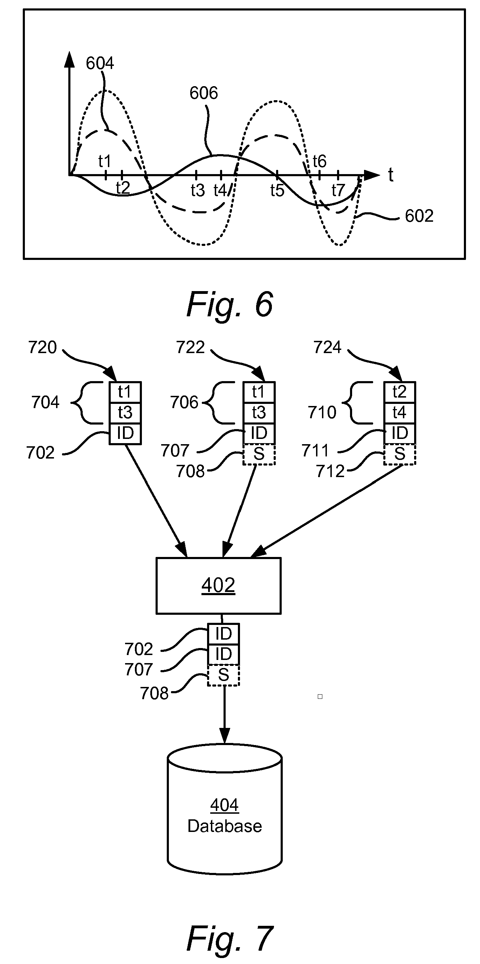

Figure 6 schematically shows different oscillating movements measured by the first/second devices.

As mentioned above, an oscillating movement is a repeating movement or a periodic motion, back and forth over a range of positions, for example measured by an accelerometer. Such movements are plotted in the graph of figure 6, where e.g. the vertical position of the first/second device is plotted on the y-axis and where the time is plotted on the x-axis. The graph comprises three different movements 602, 604, 606, thus occurring at the same time. For example, the movement 602 may originate from a first device 102, while the two other movements 604, 606 may originate from two different second devices 112.

At the x-axis, the time points for the two extremes of displacement for each single oscillating movement, i.e. one time back and forth over a range of positions, are marked t1-t7. As visible on the graph, two of the oscillation movements 602, 604 share such time points, i.e. t1, t3, t5, t7, while the remaining oscillating movement 606 has other time points, i.e. t2, t4, t6.

According to some embodiments, the microcontrollers of the first 102 and second devices 112 are arranged for time-stamping at least one of the two extremes of displacement of the first device. Such time stamps may then be included in the data sent to the third device 402. According to some embodiments, the data pertaining to an oscillating movement of the first 102 and second devices 112 comprises the time stamp of the at least one of the two extremes of displacement. Such embodiment is shown in figure 7.

In figure 7, data 720 sent from the first device (e.g. via BLE) to the third device 402 is shown. In this data 720, data 704 pertaining to an oscillating movement of the first device is included. This may be calculated by a sensor in the first device being arranged for measuring movements of the first device and for producing movement data pertaining to the measured movements (the movement 602 in figure 6), which is then sent to the microcontroller in the first device which is arranged for receiving the movement data from the sensor, and for extracting data 704 pertaining to the oscillating movement of the first device. In the example of figure 7, the microcontroller in the first device is arranged for time-stamping both of the two extremes t1, t3 of displacement of the first device and including such time stamps in the data 720 pertaining to an oscillating movement of the first device. The user ID 702 of the user wearing the first device is also included in the data 720 sent from the first device to the third device 402.

Also in figure 7, data 722 sent from the second device (e.g. via BLE) to the third device 402 is shown. In this data 722, data 706 pertaining to an oscillating movement of the second device is included. This may be calculated by a sensor in the second device being arranged for measuring movements of the second device and for producing movement data pertaining to the measured movements (the movement 604 in figure 6), which is then sent to the microcontroller in the second device which is arranged for receiving the movement data from the sensor, and for extracting data 706 pertaining to the oscillating movement of the second device. In the example of figure 7, the microcontroller in the second device is arranged for time-stamping both of the two extremes t1, t3 of displacement of the second device and including such time stamps in the data 722 pertaining to an oscillating movement of the second device. The ID 707 of the equipment onto which the second device is attached is included in the data 722 sent from the second device (e.g. via BLE) to the third device 402. According to some embodiments, also a current setting 708 of the exercise equipment onto which the second device is attached is included in the data 722 pertaining to an oscillating movement of the second device. Such setting 708 may include e.g. a weight setting of an exercise machine onto which the second device is attached, the weight of the barbell onto which the second device is attached or a resistance setting of rowing machine onto which the second device is attached.

In a similar way, data 724 is extracted from the measured movements (the movement 606 in figure 6) of a further second device.

The third device (e.g. a server) 402 which is connected to a database 404 is thus arranged for receiving the data 704 pertaining to an oscillating movement of the first device, the user ID 702, the equipment ID 707, and the data 706 pertaining to an oscillating movement of the second device. Optionally, the third device 402 is further arranged for receiving data 708 pertaining to a current setting of the exercise equipment onto which the second device is attached.

The third device 402 may at any given moment receive data from a plurality of first and/or second devices. In figure 7, the third device is also receiving the data 710 pertaining to an oscillating movement of the further second device, the equipment ID 711, and optionally the data 712 pertaining to a current setting of the exercise equipment onto which the further second device is attached.

As understood by the person skilled in the art, the third device 402 may further receive data from more first/second devices, but this is excluded from figure 7 for the ease of description.

When receiving data from at least one first device, and at least one second device, the third device is arranged for calculating a correlation between the data pertaining to an oscillating movement of the first device and the data pertaining to an oscillating movement of the second device. The third device may be arranged for looking for e.g. the user ID 702 having a certain format to distinguish data 720 received from a first device from data 722, 724 received from a second device. Any other means for distinguishing data may be employed, such as setting a specific bit in the data as a one or a zero depending from which type of device (first or second) that it originates from.

When receiving data from more than one first and/or second devices, for example as shown in figure 7, the third device 402 may be arranged to calculate a correlation for all possible combinations of data from a first device and data from a second device. According to other embodiments, the third device may be arranged to stop the correlation calculation as soon as a calculated correlation that exceeds the matching threshold is found.

In case a fourth device is included in the system for linking oscillating movements of an exercise equipment to a user of the exercise equipment in a database as shown in figure 5, and the fourth device is arranged for transmitting the ID of gym where it is located to the third device, the third device may use this ID when determining between which of the received data a correlation should be calculated as described above.

For example, in the example of figure 7, the calculation of the correlation may comprise comparing the first and second time stamps 704 of the data 720 received from the first device with the first and second time stamps of the two data 722, 724 received from the second devices, and if they match for one of the two second devices (possibly within some predetermined difference such as 0.05 sec, 0,1 sec, 0.5 sec etc), the calculated correlation is determined by the third device 402 to exceed the threshold. So in figure 7, the correlation between the data 704 pertaining to an oscillating movement of the first device and the data 706 pertaining to an oscillating movement of the second device is determined to exceed the threshold while the correlation between the data 704 pertaining to an oscillating movement of the first device and the data 7010 pertaining to an oscillating movement of the further second device is determined to not exceed the threshold.

Subsequent to the correlation calculation, any match (i.e. a correlation exceeding the threshold) between oscillating movements of a first device and a second device is linked and stored in the database. For example, using the received ID of the first device and the received equipment ID, the third device may increase a counter in a data entry which belongs to the user ID 702 and the exercise equipment having the equipment ID 707. Incrementing a counter means that repetition is counted. By including the current setting of the exercise equipment onto which the second device is attached, this can be included in the stored registry of the exercise performed by the user having the user ID 702 on the exercise equipment having the equipment ID 707, e.g. user X has performed Y repetition on machine Z with the weight N.

In the example of figure 7, only the time stamp of the at least one of the two extremes of displacement of an oscillating movement is included in the data 720, 722, 724 sent from the first/second devices to the third device. This reduced the amount of data sent to the third device, which also reduces the power consumption of the first/second device. However, it should be noted that this is just an example embodiment. According to some embodiments, the microcontrollers of the first/second devices are arranged for extracting data pertaining to a single oscillating movement from the movement data (e.g. movement data 602, 604, 606 in figure 6) from the sensors of the respective device, and using this data as the data pertaining to an oscillating movement of the respective device. In other words, instead of just sending the time stamps of one or both extremes of displacement of an oscillating movement, the first/second device may be arranged to extract accelerometer data pertaining to a single oscillating movement from the movement data of the device measured by the accelerometer, and include the accelerometer data of the complete single oscillating movement in the data sent to the third device. This may increase the possibility to detect matching movements between a first and a second device at the third device.

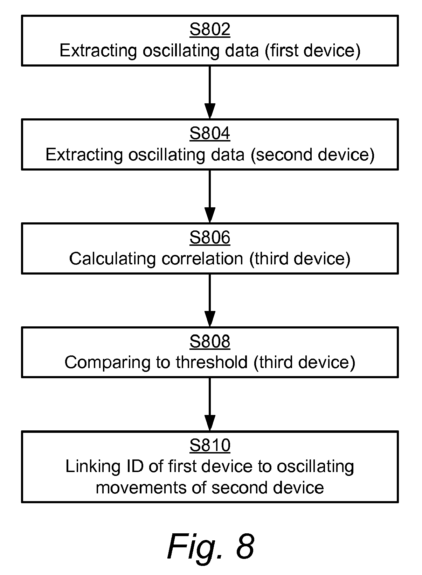

Figure 8 shows a method for linking oscillating movements of an exercise equipment to a user of the exercise equipment in a database according to some embodiments. The method being performed in a system as described above (e.g. in figure 4-5), i.e. comprising a first device adapted to be attached to a user and comprising a user ID, a second device adapted to be attached to the exercise equipment and comprising an equipment ID, and a third device connected to the database.

The method comprises the steps of:
- extracting S802, by the first device, data pertaining to an oscillating movement of the first device,
- extracting S804, by the second device, data pertaining to an oscillating movement of the second device,
- calculating S806, by the third device, a correlation between said data pertaining to an oscillating movement of the first device and said data pertaining to an oscillating movement of the second device,
- comparing S808, by the third device, the calculated correlation to a first threshold, and,
- if the calculated correlation exceeds the first threshold, linking S810, by the third device, said oscillating movement of the exercise equipment to the user ID in the database using the equipment ID.

The devices (e.g. the first, second, third and/or fourth device) and methods disclosed hereinabove may be implemented as software, firmware, hardware or a combination thereof. In a hardware implementation, the division of tasks between functional units or components referred to in the above description does not necessarily correspond to the division into physical units; to the contrary, one physical component may have multiple functionalities, and one task may be carried out by several physical components in cooperation. Certain components or all components may be implemented as software executed by a digital signal processor, microprocessor or microcontroller, or be implemented as hardware or as an application-specific integrated circuit. Such software may be distributed on computer readable media, which may comprise computer storage media (or non-transitory media) and communication media (or transitory media). As is well known to a person skilled in the art, the term computer storage media includes both volatile and nonvolatile, removable and non-removable media implemented in any method or technology for storage of information such as computer readable instructions, data structures, program modules or other data. Computer storage media includes, but is not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other medium which can be used to store the desired information and which can be accessed by a computer.

## Claims

1. A system (400, 500) for linking oscillating movements of an exercise equipment (110) to a user (100) of the exercise equipment having an equipment ID in a database (404), the system comprising:
a first device (102, 102a-z) adapted to be attached to the user, the first device comprising a user ID (702), the first device being arranged for extracting (S802) data (704) pertaining to an oscillating movement of the first device,
a second device (112, 112a-z) adapted to be attached to the exercise equipment, the second device comprising the equipment ID (707, 711), the second device being arranged for extracting (S804) data (706, 710) pertaining to an oscillating movement of the second device,
a third device (402) connected to the database and arranged for:
receiving said data pertaining to an oscillating movement of the first device, the user ID, the equipment ID and said data pertaining to an oscillating movement of the second device,
calculating (S806) a correlation between said data pertaining to an oscillating movement of the first device and said data pertaining to an oscillating movement of the second device, comparing (S808) the calculated correlation to a first threshold, and if the calculated correlation exceeds the first threshold, linking (S810) said oscillating movement of the exercise equipment to the user ID in the database using the equipment ID.

2. The system according to claim 1,
wherein the first device comprising:
a first sensor (202) arranged for measuring movements of the first device and for producing movement data pertaining to the measured movements,
a first microcontroller (208) arranged for receiving the movement data from the first sensor, and for extracting data pertaining to the oscillating movement of the first device, and
wherein the second device comprising:
a second sensor (202) arranged for measuring movement of the second device
a second microcontroller (208) arranged for receiving movement data from the second sensor, and for extracting data pertaining to the oscillating movement of the second device.

3. The system according to claim 2, wherein the second sensor is a battery driven accelerometer having a first and a second state, wherein the first state is a low energy state wherein the accelerometer is arranged for detecting a movement exceeding a threshold and in a response thereto configuring itself in a second state wherein the accelerometer is arranged for measuring movements of the second device and for producing movement data pertaining to the measured movements.

4. The system according to any one of claims 2-3,
wherein the oscillating movement of the first device and the second device comprises two extremes of displacement,
wherein the first microcontroller is arranged for time-stamping (t1-t7) at least one of the two extremes of displacement of the first device and wherein said data pertaining to an oscillating movement of the first device comprises the time stamp of the at least one of the two extremes of displacement of the first device, and
wherein the second microcontroller is arranged for time-stamping (t1-t7) at least one of the two extremes of displacement of the second device and wherein said data pertaining to an oscillating movement of the second device comprises the time stamp of the at least one of the two extremes of displacement of the second device.

5. The system according to any one of claims 2-3, wherein the first and second sensors are accelerometers and wherein the movement data from the first and second sensors comprises data from the respective accelerometer, wherein first and second microcontroller are arranged for extracting data pertaining to a single oscillating movement from the movement data from the first and second sensors respectively, and using this data as the data pertaining to an oscillating movement of the first and second device respectively.

6. The system according to any one of claims 1-5, wherein the third device is a part of the first or the second device.

7. The system according to any one of claims 1-5, wherein the third device is a separate device from the first and the second device.

8. The system according to claim 7, wherein the system further comprises a fourth device (502a-b) being wirelessly connected to the first and second device, and further being connected to the third device,
wherein the fourth device being arranged for receiving said data pertaining to an oscillating movement of the first device, the user ID, the equipment ID, and said data pertaining to an oscillating movement of the second device and for transmitting said data pertaining to an oscillating movement of the first device, the user ID, the equipment ID, and said data pertaining to an oscillating movement of the second device to the third device.

9. The system according to claim 8, wherein the fourth device comprises an ID of a gym wherein the exercise equipment is located, wherein the fourth device is further arranged for transmitting the ID of the gym to the third device.

10. The system according to any one of claims 1-9, wherein the first and second device comprises a Bluetooth Low Energy, BLE, radio (204) arranged for transmitting the data pertaining to an oscillating movement of the first and second device respectively.

11. The system according to any one of claims 1-10, wherein the third device is arrange for linking said oscillating movement of the exercise equipment to the user ID in the database by incrementing a counter.

12. The system according to any one of claims 1-11, wherein the third device is further arranged for receiving data pertaining to a current setting of the exercise equipment, and for adding said data pertaining to a current setting (708, 712) of the exercise equipment to the database when linking said oscillating movement of the exercise equipment to the user ID in the database.

13. A method for linking oscillating movements of an exercise equipment (110) to a user (100) of the exercise equipment in a database (404), the method being performed in a system (400, 500) comprising a first device (102, 102a-z) adapted to be attached to a user and comprising a user ID, a second device adapted to be attached to the exercise equipment and comprising an equipment ID of the exercise equipment, and a third device connected to the database, the method comprising the steps of:
extracting (S802), by the first device, data pertaining to an oscillating movement of the first device,
extracting (S804), by the second device, data pertaining to an oscillating movement of the second device,
calculating (S806), by the third device, a correlation between said data pertaining to an oscillating movement of the first device and said data pertaining to an oscillating movement of the second device,
comparing (S808), by the third device, the calculated correlation to a first threshold, and,
if the calculated correlation exceeds the first threshold, linking (S810), by the third device, said oscillating movement of the exercise equipment to the user ID in the database using the equipment ID.

14. The method of claim 13, wherein the oscillating movement of the first device and the second device comprises two extremes of displacement,
wherein said data pertaining to an oscillating movement of the first device comprises the time stamp of the at least one of the two extremes of displacement of the first device, and
wherein said data pertaining to an oscillating movement of the second device comprises the time stamp of the at least one of the two extremes of displacement of the second device.

15. The method according to claim 13,
wherein the oscillating movement of the first device comprises data from an accelerometer of the first device pertaining to a single oscillating movement of the first device as detected by the accelerometer of the first device,
wherein the oscillating movement of the second device comprises data from an accelerometer of the second device pertaining to a single oscillating movement of the second device as detected by the accelerometer of the second device.
